# EUROPEAN PATENT SPECIFICATION

(11) **EP 1 628 591 B1**
(45) Date of publication and mention of the grant of the patent: **15.09.2010**
(21) Application number: 04735840.3
(22) Date of filing: 02.06.2004
(51) Int. Cl.: A61B 19/08

(54) **DRAPE PRODUCT FOR SURGICAL INTERVENTIONS**
TUCH FÜR CHIRURGISCHE EINGRIFFE
CHAMP OPERATOIRE POUR INTERVENTIONS CHIRURGICALES

(30) Priority: 02.06.2003 SE 0301589
(43) Date of publication of application: 01.03.2006
(73) Proprietor: Mölnlycke Health Care AB, 402 52 Göteborg (SE)
(72) Inventor: RAUK BERGSTRÖM, Tina, S-421 65 Västra Frölunda (SE); KARLSSON, Fredrik, S-43833 LANDVETTER (SE)
(74) Representative: Hyltner, Jan-Olof
(86) International application number: PCT/SE2004/000849
(87) International publication number: WO 2004/105627

(56) References cited:
- US-A- 4 570 628
- US-A- 5 709 220
- US-A- 5 813 408
- US-B1- 6 269 815

## Description

### TECHNICAL FIELD

The present invention relates to a drape product for surgical interventions, comprising an opening intended to be arranged around an operating site on a patient, which drape product, during use, forms a barrier between the operating site and that part of the patient's body lying outside the operating site.

### BACKGROUND TO THE INVENTION

In certain surgical interventions, e.g. gynecological interventions, such as prolapse operations, the surgeon moves the external labia aside and secures them in this position with sutures. The sutures are secured either in the patient or in the drape product which surrounds the operating site. Both these solutions have disadvantages of different types. If the sutures are secured in the patient, this causes the patient pain and discomfort after the operation, and this can turn out to be more problematic than the sequelae of the actual surgical intervention. If the sutures are secured instead in the drape product, the barrier which the drape product forms between the operating site and that part of the patient's body lying outside the operatiing site is broken. This barrier is intended on the one hand to ensure that bacteria and the like from the patient's body do not contaminate the operating site, and to ensure that blood, bacteria and such like from the operating site do not reach the parts of the patient's body lying outside the operating site and do not contaminate the operating table and other operating equipment. Here, drape product signifies surgical sheets, systems of surgical sheets or the like.

The present invention aims to overcome these disadvantages. US 4,570,628 shows a drape according to the preamble of claim 1, having separate holding strips for medical instruments through which strips a suture can be passed.

### DISCLOSURE OF THE INVENTION

According to the invention, this object is achieved by means of a drape product for surgical interventions:, comprising an opening intended to be arranged around an operating site on a patient, which drape product, during use, forms a barrier between the operating site and that part of a patient's body lying outside the operating site, the drape product comprises at least one member for receiving and/or securing sutures or the like, through which member a suture or the like can be passed without damaging or destroying the barrier between the operating site (O) and that part of a patient's body lying outside the operating site, the members for receiving and/or securing sutures or the like consist of upwardly protecting folds in the product.

In a preferred embodiment, members for receiving and/or securing sutures or the like are arranged on at least two sides of the opening of the product in such a way that the flow of fluid from the operating site is not impeded by these members. Each member is situated at a distance of 15 - 150 mm from the nearest edge of the opening of the product.

In an advantageous variant, the drape product consists of a surgical sheet for gynecological operations, with an operation opening which, during use of the sheet, has an upper edge, a lower edge, and two opposite side edges, and members for receiving and/or securing sutures or the like are arranged on both sides of the operation opening.

### DESCRIPTION OF THE FIGURES

The invention will now be described with reference to the attached figures, in which:
Fig. 1 shows a diagrammatic plan view, from above, of a gynecological sheet according to a first embodiment of the invention,
Fig. 2 shows a transverse section along the line II-II in Figure 1,
Figure 3 shows a member for receiving and/or securing sutures not according to the invention, and
Fig. 4 shows diagrammatically a drape system comprising four surgical drapes which are arranged around an operation opening and are provided with members for receiving and/or securing sutures according to the invention.

### DESCRIPTION OF EMBODIMENTS

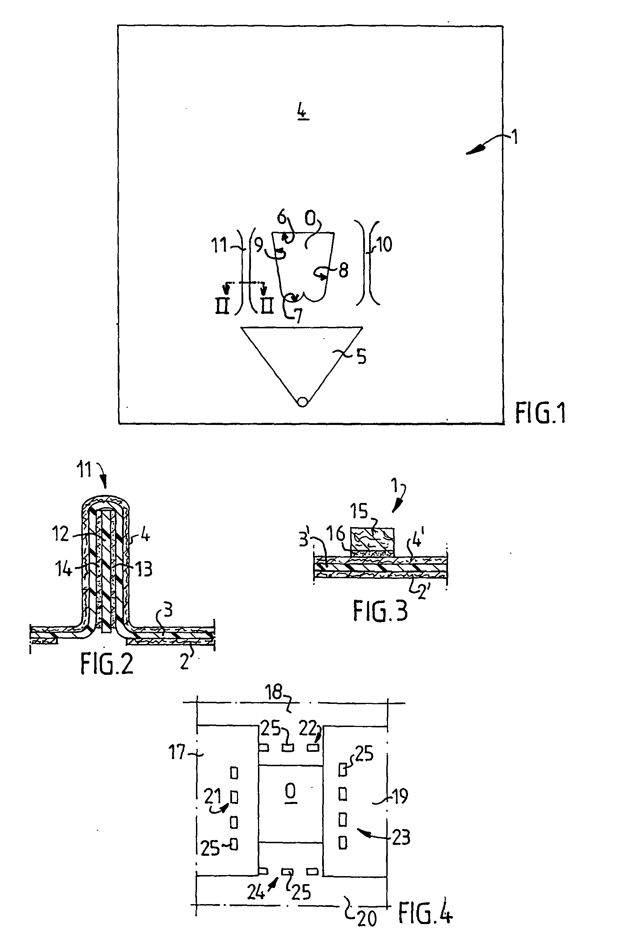

Figures 1 and 2 show diagrammatic views of a surgical sheet 1 for gynecological operations according to a preferred embodiment of the invention. The sheet 1 comprises an intermediate plastic layer 3, for example of polyethylene, which ensures the sheet's barrier effect, and an upper layer 4 of absorbent material, for example nonwoven. The sheet 1 can also have a lower comfort-enhancing layer 2 which can consist of cellulose or nonwoven fabric. The sheet also has an operation opening O and a pouch 5 for collecting fluid running from the operating site. The operation opening has an upper edge 6, a lower edge 7, which during use of the sheet is located at a lower level than the upper edge 6, and two side edges 8, 9.

The sheet 1 also has two upwardly projecting folds 10, 11 which extend along the side edges 8, 9 on both sides of the opening O. As can be seen from Figure 2, the insides of those parts of the sheet 1 forming the fold 11 are connected to one another by means of a tape 12 provided with adhesive layers 13, 14 on both sides. Inside the area for the double-sided adhesive tape 12, a needle can be passed through the fold 11 without the hole formed in the fold damaging or destroying the sterile barrier which the sheet 1 forms between the operating site O and that part of the patient's body lying outside the operating site. For this reason, it is important that the insides of the fold are connected to one another along the whole length of the fold and the tape 12 extends uninterruptedly along the full length of the fold. It should be noted here that, at the transitions between the ends of the fold and outside plane areas of the sheet, said sheet will have upwardly projecting parts through which needles cannot be passed without damaging or destroying the barrier effect of the sheet. Moreover, the optional comfort-enhancing layer 2 will not extend into the area of the folds.

The folds 10, 11 are preferably placed at a distance of 15 - 150 mm from the respective side edge 8, 9 and thus they lie to a large extent outside the natural passage of the flow of fluid from the operating site O.

The insides of the fold can of course be connected to one another in another way than by means of double-sided adhesive tape, for example by means of an adhesive layer or a thermal weld connection.

The invention can of course also be applied to drape products of another structure or type than the gynecological sheet described here, for example for two-layer or one-layer surgical sheets or drapes and for sheets intended for other types of operations, for example operations on the head.

Figure 3 shows a sheet not according to the invention comprising members for receiving and/or securing sutures or the like. These members consist of separate elongate material sections 15 secured to the sheet 1' by means of an adhesive layer 16. The material section 15 will consist of material which it is easy to sew in, and can for example consist of textile material, nonwoven fabric, plastic material, foamed plastic or so-called hook-and-loop material.

Figure 4 shows an embodiment of the invention. In this embodiment, four surgical sheets 17-20 are placed on a patient so that an operation opening O is formed. Extending along the edge of each sheet 17-20 forming an edge of the operation opening O, there is a row 21, 22, 23, 24 of separate, individual material sections 25 at a distance of 15 - 150 mm from the edge. By virtue of the fact that there are spaces between the material sections, fluid can flow freely from the operating site O without being impeded by the rows of members 25 for receiving and/or securing sutures, irrespective of the direction of flow of the fluid. It is also conceivable to arrange rows of folds along an edge of the operation opening, but this is not preferred.

To make it easier to insert a needle and secure a suture or the like, the folds 10, 11 expediently have a height which is greater than 15 mm and the material sections expediently have a minimum height of 5 mm and a minimum securing length of 10 mm. Securing length here means the maximum value of the height or width of the material section. Such dimensioning ensures that the surgeon has access to a horizontal or vertical sewing surface with sufficient area for a suture to be easily secured in the material section, without risk of the needle making a hole on the drape material on which the material sections are secured.

The embodiments described above can of course be modified within the scope of the invention. For example, the folds 10, 11 can be formed in some other way, for example folded in two to make them stiffer, which can be desirable if one wishes to prevent their being folded in towards the plane of the drape by the tension in sutures secured to them. Moreover, the members for receiving and/or securing sutures do not need to extend in a straight line or parallel to the edges of the operation opening. For example, a stiffening effect can be obtained by elongate members being routed in a sinusoidal or zigzag shape. The invention will therefore only be limited by the content of the attached patent claims.

## Claims

1. A drape product (1; 1'; 17-20) for surgical interventions, comprising an opening (O) intended to be arranged around an operating site on a patient, which drape product, during use, forms a barrier between the operating site and that part of a patient's body lying outside the operating site, the drape product comprises at least one member (10, 11; 25) for receiving and/or securing sutures or the like, through which member a suture or the like can be passed without damaging or destroying the barrier between the operating site (O) and that part of a patient's body lying outside the operating site, **characterized in that** the members (10, 11) for receiving and/or securing sutures or the like consist of upwardly projecting folds in the product (1).

2. The drape product as claimed in claim 1, **characterized in that** members (10, 11; 25) for receiving and/or securing sutures or the like are arranged on at least two sides of the opening (O) of the product in such a way that the flow of fluid from the operating site is not impeded by these members.

3. The drape product as claimed in claim 1 or 2, **characterized in that** each member (10, 11; 25) is situated at a distance of 15 - 150 mm from the nearest edge of the opening of the product.

4. The drape product as claimed in claim 1, 2 or 3, **characterized in that** the drape product consists of a surgical sheet (1) for gynecological operations with an operation opening (O) which, during use of the sheet, has an upper edge (6), a lower edge (7), and two opposite side edges (8, 9), and members (10, 11) for receiving and/or securing sutures or the like are arranged on both sides of the operation opening (O).

## Patentansprüche

1. Vorhangprodukt (1; 1'; 17-20) für chirurgische Eingriffe, umfassend eine Öffnung (O), die dazu gedacht ist, um eine Operationslokalisation an einem Patienten angeordnet zu werden, welches Vorhangprodukt während der Verwendung eine Barriere zwischen der Operationslokalisation und dem Teil des Körpers eines Patienten bildet, der außerhalb der Operationslokalisation liegt, wobei das Vorhangprodukt zumindest ein Element (10, 11; 25) zum Aufnehmen und/oder Befestigen von Fäden oder dergleichen umfasst, durch welches Element ein Faden oder dergleichen hindurchgeführt werden kann, ohne die Barriere zwischen der Operationslokalisation (0) und dem Teil des Körpers eines Patienten, der außerhalb der Operationslokalisation liegt, zu zerstören, **dadurch gekennzeichnet, dass** die Elemente (10, 11) zum Aufnehmen und/oder Befestigen von Fäden oder dergleichen aus nach oben vorstehenden Falten in dem Produkt (1) bestehen.

2. Vorhangprodukt nach Anspruch 1, **dadurch gekennzeichnet, dass** Elemente (10, 11; 25) zum Aufnehmen und/oder Befestigen von Fäden oder dergleichen an zumindest zwei Seiten der Öffnung (0) des Produkts derart angeordnet sind, dass die Strömung eines Fluids von der Operationslokalisation durch diese Elemente nicht gehemmt wird.

3. Vorhangprodukt nach Anspruch 1 oder 2, **dadurch gekennzeichnet, dass** jedes Element (10, 11; 25) in einem Abstand von 15 - 150 mm von der nächsten Kante der Öffnung des Produkts angeordnet ist.

4. Vorhangprodukt nach Anspruch 1, 2 oder 3, **dadurch gekennzeichnet, dass** das Vorhangprodukt aus einem chirurgischen Laken (1) für gynäkologische Operationen mit einer Operationsöffnung (0) besteht, die während der Verwendung des Lakens eine obere Kante (6), eine untere Kante (7) und zwei gegenüberliegende Seitenkanten (8, 9) aufweist, und Elemente (10, 11) zum Aufnehmen und/oder Befestigen von Fäden oder dergleichen auf beiden Seiten der Operationsöffnung (0) angeordnet sind.

## Revendications

1. Produit de drap opératoire (1 ; 1'; 17-20) pour des interventions chirurgicales, comprenant une ouverture (O) destinée à être agencée autour d'un site opératoire sur un patient, lequel produit de drap opératoire, pendant l'utilisation, forme une barrière entre le site opératoire et cette partie du corps d'un patient qui se trouve à l'extérieur du site opératoire, le produit de drap opératoire comprenant au moins un élément (10, 11 ; 25) destiné à recevoir et/ou fixer des sutures ou similaires, à travers lequel élément, on peut faire passer une suture ou similaire sans endommager ni détruire la barrière entre le site opératoire (O) et cette partie du corps d'un patient qui se trouve à l'extérieur du site opératoire, **caractérisé en ce que** les éléments (10, 11) destinés à recevoir et/ou fixer les sutures ou similaires se composent de plis faisant saillie vers le haut dans le produit (1).

2. Produit de drap opératoire selon la revendication 1, **caractérisé en ce que** des éléments (10, 11 ; 25) destiné à recevoir et/ou fixer des sutures ou similaires sont agencés sur au moins deux côtés de l'ouverture (O) du produit de sorte que l'écoulement du fluide provenant du site opératoire ne soit pas gêné par ces éléments.

3. Produit de drap opératoire selon la revendication 1 ou 2, **caractérisé en ce que** chaque élément (10, 11 ; 25) est situé à une distance de 15-150 mm du bord le plus proche de l'ouverture du produit.

4. Produit de drap opératoire selon la revendication 1, 2 ou 3, **caractérisé en ce que** le produit de drap opératoire se compose d'un drap chirurgical (1) pour des opérations gynécologiques avec une ouverture d'opération (O) qui, pendant l'utilisation du drap, a un bord supérieur (6), un bord inférieur (7) et deux bords latéraux opposés (8, 9), et des éléments (10, 11) destinés à recevoir et/ou fixer des sutures ou similaires sont agencés des deux côtés de l'ouverture d'opération (O).
